# EUROPEAN PATENT APPLICATION

(11) **EP 4 260 827 A1**
(43) Date of publication of application: **18.10.2023**
(21) Application number: 22766898.5
(22) Date of filing: 28.02.2022
(51) Int. Cl.: A61B 17/94, A61B 18/14

(54) **ENDOSCOPIC TREATMENT IMPLEMENT**

(30) Priority: 12.03.2021 JP 2021040175
(71) Applicant: Hoya Corporation, Tokyo 160-8347 (JP)
(72) Inventor: SUGITA Noriyuki, Tokyo 160-8347 (JP); OHATA Ken, Tokyo 162-0842 (JP)
(74) Representative: Lucke, Andreas
(86) International application number: PCT/JP2022/008379
(87) International publication number: WO 2022/190939

(57) **Abstract**

An endoscopic treatment tool is configured to include a tubular portion; a rod-shaped member that is disposed to protrude from a distal end surface of the tubular portion, and has a variable protrusion amount from the distal end surface in response to operations of an operation unit; a first connection port to which a tool for water supply or suction is connectable; a first distal end opening that is formed in a distal end surface of the rod-shaped member; a first channel that is formed in the tubular portion to extend in an axial direction of the tubular portion, and has a distal end communicating with the first distal end opening and a proximal end communicating with the first connection port; a second connection port to which the tool is connectable; a second distal end opening that is formed in the distal end surface of the tubular portion; and a second channel that is formed to extend in the axial direction within the tubular portion, and has a distal end communicating with the second distal end opening and a proximal end communicating with the second connection port.

## Description

### Technical Field

The present invention relates to an endoscopic treatment tool.

### Background Art

Endoscopic submucosal dissection (hereinafter, referred to as "ESD") is known as a method capable of reliably and collectively excising a wide range of lesions such as early esophageal cancer, early gastric cancer, and early colorectal cancer by using an endoscope. An ESD procedure includes a treatment of marking an excision range of a lesion (a marking treatment), locally injecting a drug solution into a submucosa to raise a mucosal lesion (a local injection treatment), incising a periphery of the mucosal lesion according to the marking, then exfoliating the submucosa (an incision/exfoliation treatment), and stopping bleeding occurred in an exfoliated ulcer surface, as well as during the incision and the exfoliation (a hemostatic treatment). In this type of procedure, an endoscopic treatment tool including, for example, a nozzle for excising a mucous membrane or the like by applying a high-frequency current is used (e.g., with reference to Patent Literature 1).

### Citation List

### Patent Literature

Patent Literature 1: JP 2019-213883 A

### Summary of Invention

### Technical Problem

In a procedure using this type of endoscopic treatment tool, for example, water may be supplied for local injection into a submucosa or specifying a hemostatic site during bleeding, or blood or mucus may be aspirated to secure a surgical field. In a conventional endoscopic treatment tool exemplified in Patent Literature 1, in a case of performing water supply, when a suction pump is connected to the endoscopic treatment tool, it is necessary to connect a water supply pump to the endoscopic treatment tool after removing the suction pump, and in a case of performing suction, when the water supply pump is connected to the endoscopic treatment tool, it is necessary to connect the suction pump to the endoscopic treatment tool after removing the water supply pump. Such exchange work of the pumps causes a decrease in procedure efficiency.

The present invention has been made in view of the above circumstances, and an object of the present invention is to provide an endoscopic treatment tool capable of improving the procedure efficiency.

### Solution to Problem

The endoscopic treatment tool according to one embodiment of the present invention includes a tubular portion; a rod-shaped member that is disposed to protrude from a distal end surface of the tubular portion, and has a variable protrusion amount from the distal end surface in response to operations of an operation unit; a first connection port to which a tool for water supply or suction is connectable; a first distal end opening that is formed in a distal end surface of the rod-shaped member; a first channel that is formed in the tubular portion to extend in an axial direction of the tubular portion, and has a distal end communicating with the first distal end opening and a proximal end communicating with the first connection port; a second connection port to which the tool is connectable; a second distal end opening that is formed in the distal end surface of the tubular portion; and a second channel that is formed to extend in the axial direction within the tubular portion, and has a distal end communicating with the second distal end opening and a proximal end communicating with the second connection port.

The endoscopic treatment tool according to one embodiment of the present invention may be configured to further include a tube communicating with a through passage formed in the rod-shaped member. In this case, the first distal end opening is an opening of the through passage formed in the distal end surface of the rod-shaped member. Further, the first channel includes the through passage and the tube.

The endoscopic treatment tool according to one embodiment of the present invention may be configured to have a proximal end of the rod-shaped member inserted into a distal end of the tube such that the through passage communicates with the tube. In this case, as the tube moves forward and backward in the axial direction within the tubular portion in response to the operations of the operation unit, the rod-shaped member also moves forward and backward in the axial direction, and the protrusion amount of the rod-shaped member from the distal end surface of the tubular portion changes.

In a front view of the distal end surface of the tubular portion, the rod-shaped member may be configured to be located in a central region of the distal end surface of the tubular portion, and the second distal end opening may be configured to be located in a peripheral region of the distal end surface of the tubular portion located outside the central region.

Further, a plurality of the second distal end openings may be formed in the distal end surface of the tubular portion. In this case, the plurality of the second distal end openings are located side by side at an equal pitch around the rod-shaped member in the front view.

Further, the tubular portion may be configured to include a sheath and a cap-shaped member attached to the sheath to close a distal end opening of the sheath. In this case, the cap-shaped member includes a first through passage that slidably supports the rod-shaped member in the axial direction, has an opening on a proximal end side communicating with the sheath and an opening on a distal end side formed in a distal end surface of the cap-shaped member, and penetrates the cap-shaped member; and a second through passage that is formed to extend in the axial direction around the first through passage, and penetrates the cap-shaped member. The second through passage forms a part of the second channel. The opening on the distal end side of the second through passage is the second distal end opening, and is formed in the distal end surface of the cap-shaped member.

Further, the tubular portion may be configured to include a support passage that slidably supports the rod-shaped member in the axial direction. In this case, the support passage forms a part of the second channel.

Further, the tubular portion may be configured to include a sheath and a cap-shaped member attached to the sheath to close a distal end opening of the sheath. In this case, the support passage is a third through passage that penetrates the cap-shaped member, and has an opening on the proximal end side communicating with the sheath and an opening on the distal end side formed in the distal end surface of the cap-shaped member. The third through passage forms a part of the second channel.

The endoscopic treatment tool according to one embodiment of the present invention may be configured such that at least a part of the first channel passes through the second channel, and a seal member securing airtightness of the second channel inside the tubular portion is disposed on an outer periphery of a portion of the first channel passing through the second channel.

The endoscopic treatment tool according to one embodiment of the present invention may be configured to further include a contact portion to which a high-frequency power supply for allowing a high-frequency current to flow is connectable, and a wire having conductivity disposed in the tubular portion. In this configuration, the rod-shaped member has conductivity. Further, the wire has a distal end electrically connected to the rod-shaped member and a proximal end electrically connected to the contact portion.

### Advantageous Effects of Invention

According to one embodiment of the present invention, provided is an endoscopic treatment tool capable of improving procedure efficiency.

### Brief Description of Drawings

Fig. 1 is a perspective view illustrating a configuration of an endoscopic treatment tool according to one embodiment of the present invention.
Fig. 2 is a side cross-sectional view illustrating a configuration of the endoscopic treatment tool according to one embodiment of the present invention.
Fig. 3 is a side cross-sectional view illustrating a configuration of the endoscopic treatment tool according to one embodiment of the present invention.
Fig. 4 is an enlarged view illustrating a part of the endoscopic treatment tool according to one embodiment of the present invention in an enlarged manner.
Fig. 5A is an enlarged view illustrating a part of the endoscopic treatment tool according to one embodiment of the present invention in an enlarged manner.
Fig. 5B is an enlarged view illustrating a part of the endoscopic treatment tool according to one embodiment of the present invention in an enlarged manner.
Fig. 6 is an enlarged view illustrating a part of the endoscopic treatment tool according to one embodiment of the present invention in an enlarged manner.
Fig. 7 is an enlarged view illustrating a part of the endoscopic treatment tool according to one embodiment of the present invention in an enlarged manner.
Fig. 8A is a perspective view of a distal end of a tubular portion of the endoscopic treatment tool according to one embodiment of the present invention.
Fig. 8B is a perspective view of the distal end of the tubular portion of the endoscopic treatment tool according to one embodiment of the present invention.
Fig. 9 is a front view of a distal end surface of the tubular portion of the endoscopic treatment tool according to one embodiment of the present invention.
Fig. 10 is a cross-sectional view illustrating a water supply channel provided in the endoscopic treatment tool according to one embodiment of the present invention.
Fig. 11 is a cross-sectional view illustrating a suction channel provided in the endoscopic treatment tool according to one embodiment of the present invention.
Fig. 12A is a perspective view of a distal end of a tubular portion of an endoscopic treatment tool according to a modification of the present invention.
Fig. 12B is a perspective view of the distal end of the tubular portion of the endoscopic treatment tool according to the modification of the present invention.
Fig. 13 is a front view of a distal end surface of the tubular portion of the endoscopic treatment tool according to the modification of the present invention.
Fig. 14 is a cross-sectional view illustrating a water supply channel provided in the endoscopic treatment tool according to the modification of the present invention.
Fig. 15 is a cross-sectional view illustrating a suction channel provided in the endoscopic treatment tool according to the modification of the present invention.

### Description of Embodiments

Hereinafter, an endoscopic treatment tool according to one embodiment of the present invention will be described with reference to the drawings.

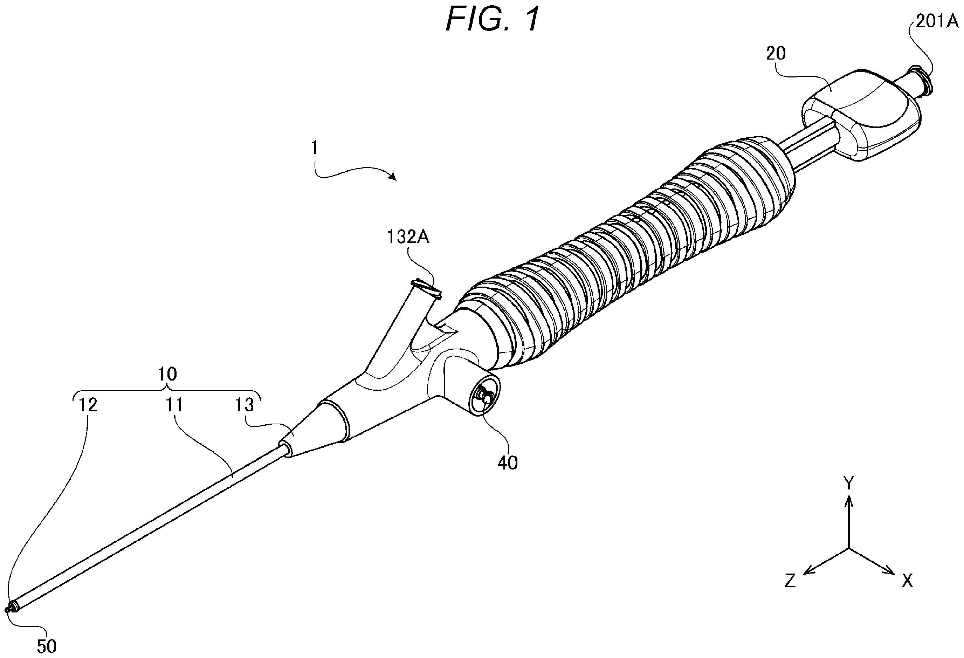

Fig. 1 is a perspective view illustrating a configuration of an endoscopic treatment tool 1 according to one embodiment of the present invention. The endoscopic treatment tool 1 includes a first channel and a second channel, and for example, can simultaneously perform one or both of water supply and suction by connecting a water supply pump to one channel and connecting a suction pump to the other channel without exchanging the pumps.

As will be described in detail later, the endoscopic treatment tool 1 includes a water supply channel 60 and a suction channel 70 as an example of the first channel and the second channel. For example, a water supply pump is connected to a proximal end of the water supply channel 60. When a cleaning liquid is supplied by the water supply pump, the cleaning liquid is ejected from the distal end of the endoscopic treatment tool 1 to the outside via the water supply channel 60. Further, for example, a suction pump is connected to a proximal end of the suction channel 70. When the suction pump is driven, attachments in a body cavity are sucked into the suction channel 70 and put into, for example, a container included in the suction pump.

As illustrated in Fig. 1, the endoscopic treatment tool 1 includes a tubular portion 10. In the following description, an axial direction (a longitudinal direction) of the tubular portion 10 is defined as a Z direction, and two directions orthogonal to the Z direction and orthogonal to each other are defined as an X direction and a Y direction. The X direction, the Y direction, and the Z direction orthogonal to each other form a right-handed system. For convenience of description, a positive side in the Z direction is also referred to as a front side, a negative side in the Z direction is also referred to as a rear side, a positive side in the Y direction is also referred to as an upper side, and a negative side in the Y direction is also referred to as a lower side, but designations of the directions are used for convenience to describe a relative positional relationship of components, and do not indicate an absolute direction.

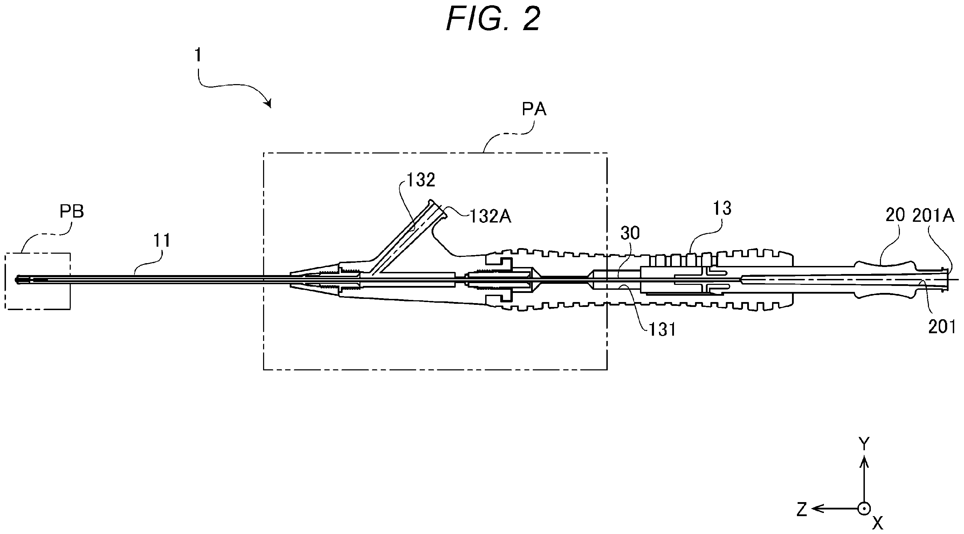

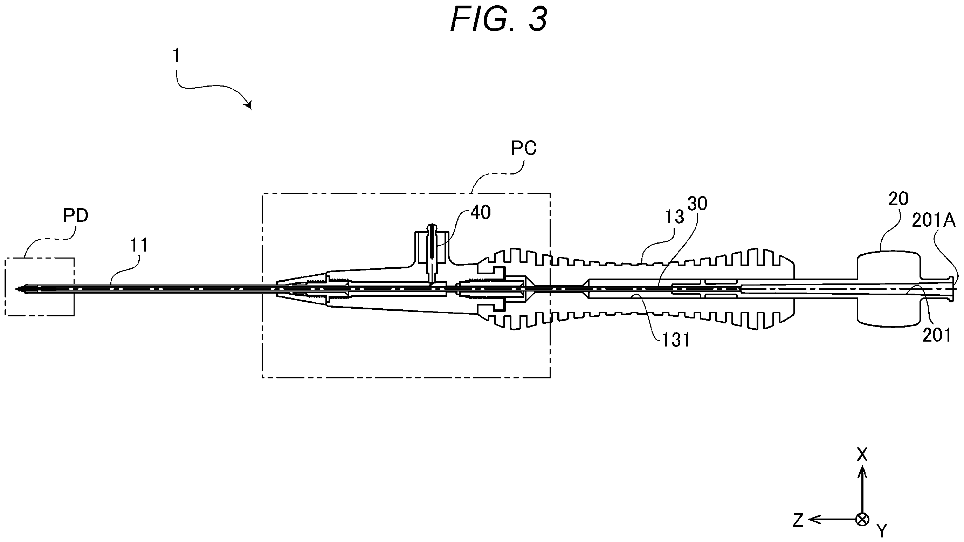

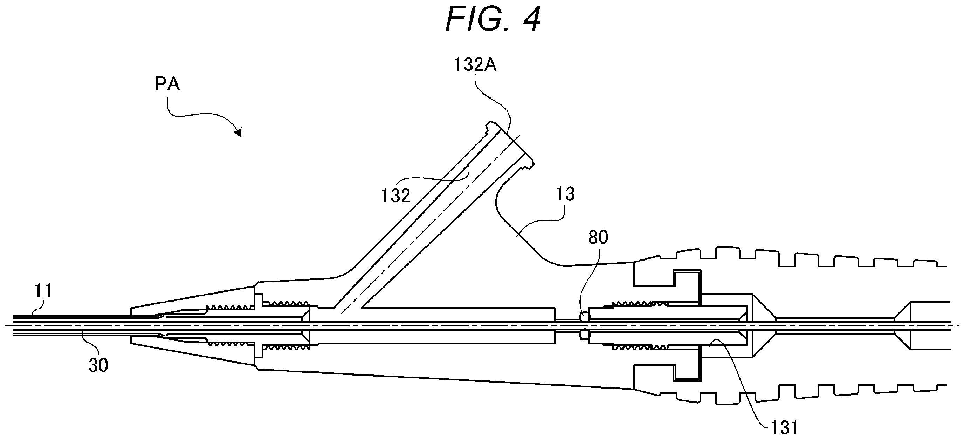

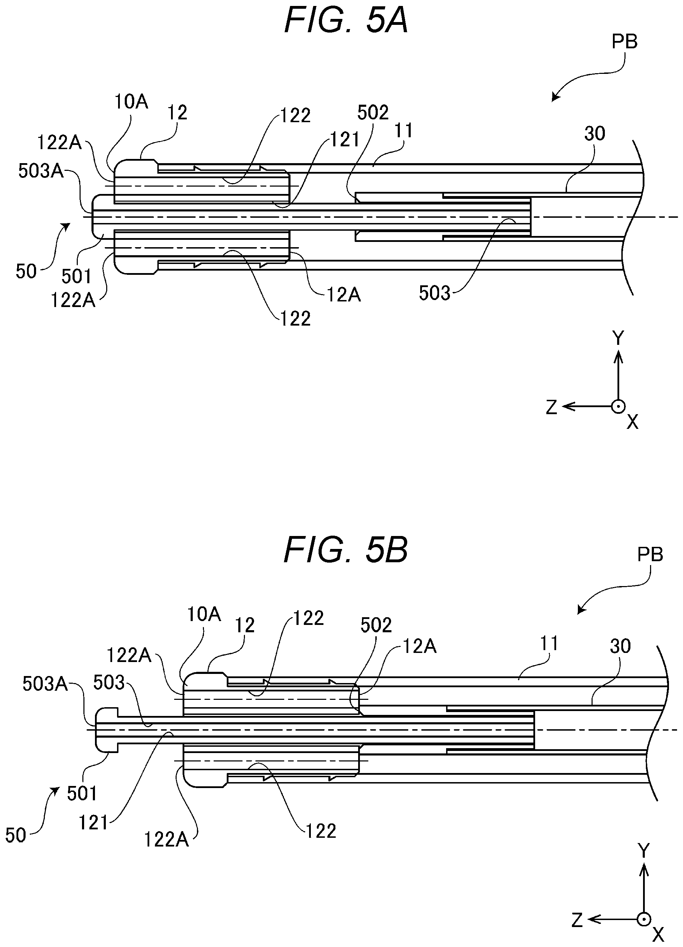

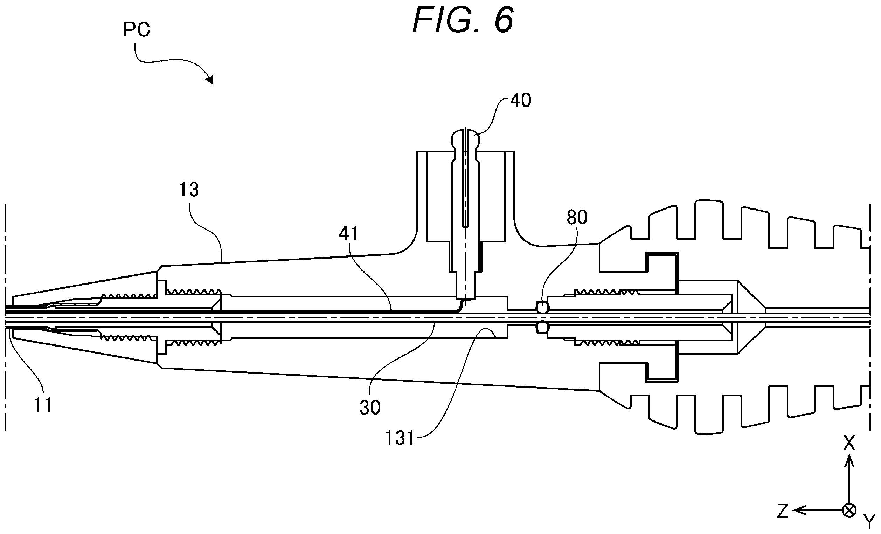

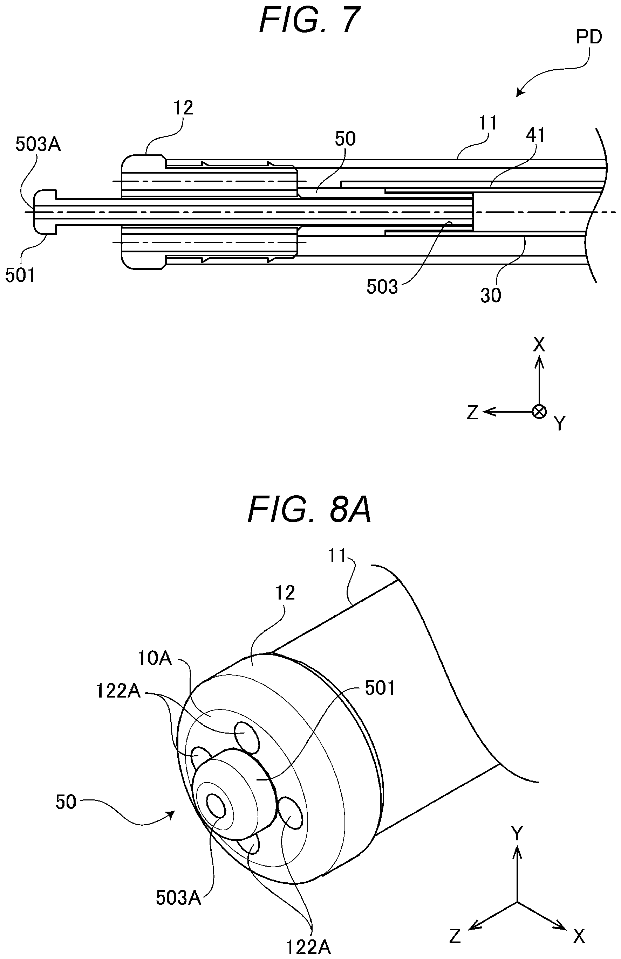

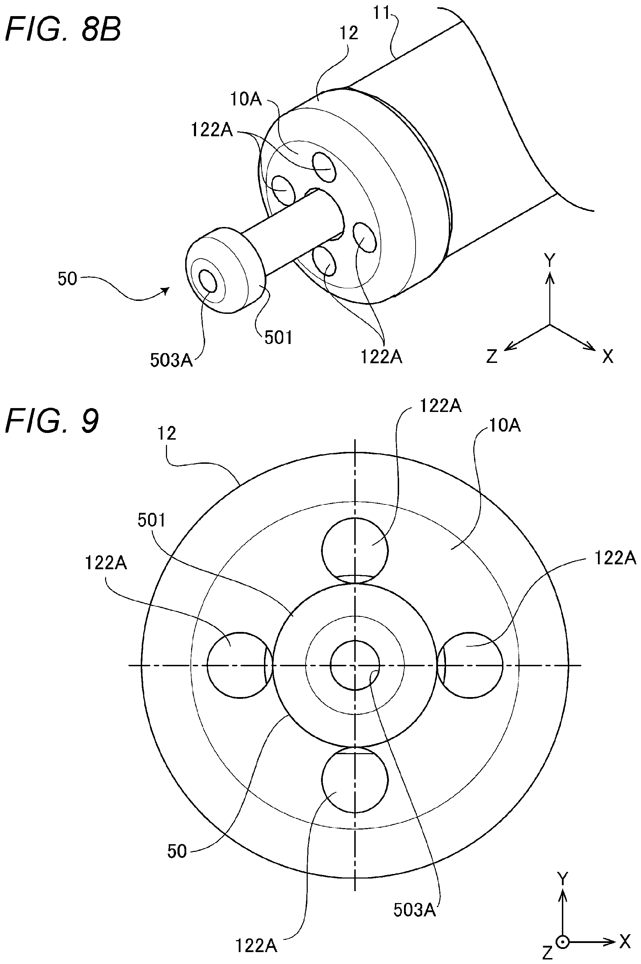

Fig. 2 and Fig. 3 are side cross-sectional views each illustrating a configuration of the endoscopic treatment tool 1 according to one embodiment of the present invention. Fig. 2 illustrates a YZ cross section including a central axis of the tubular portion 10. Fig. 3 illustrates an XZ cross section including the central axis of the tubular portion 10. Fig. 4 is an enlarged view illustrating a portion PA in Fig. 2 in an enlarged manner. Fig. 5A and Fig. 5B are enlarged views illustrating a portion PB in Fig. 2 in an enlarged manner. Fig. 6 is an enlarged view illustrating a portion PC in Fig. 3 in an enlarged manner. Fig. 7 is an enlarged view illustrating a portion PD in Fig. 3 in an enlarged manner. Fig. 8A and Fig. 8B are perspective views of a distal end of the tubular portion 10. Fig. 9 is a view when a distal end surface 10A of the tubular portion 10 is viewed from the positive side in the Z direction (in other words, a front view of the distal end surface 10A).

The tubular portion 10 includes a sheath 11 insertable into a treatment tool insertion channel of an endoscope (not illustrated), a stopper 12 fitted to a distal end opening of the sheath 11, and a housing portion 13 holding a proximal end of the sheath 11 and holding each component.

The sheath 11 is an insulating tube made of a resin having flexibility. The sheath 11 is made from, for example, polyetheretherketone (PEEK) or polytetrafluoroethylene (PTFE).

The stopper 12 is a cap-shaped member having an outer diameter substantially the same as an inner diameter of the sheath 11, and has an outer peripheral surface formed with a retaining protrusion. The stopper 12 is press-fitted into the sheath 11 from the distal end opening of the sheath 11 and fitted to an inner surface of the sheath 11. In other words, the stopper 12 is attached to the sheath 11 to close the distal end opening of the sheath 11.

The stopper 12 is an insulating member and is made of, for example, ceramics. Note that a distal end surface of the stopper 12 is the distal end surface 10A of the tubular portion 10. Therefore, the distal end surface 10A of the tubular portion 10 may also be referred to as the distal end surface 10A of the stopper 12.

The stopper 12 is formed with a through passage 121 extending in the Z direction to penetrate a center part of the stopper. A plurality of through passages 122 extending in parallel with the through passage 121 to penetrate the stopper 12 are formed around the through passage 121. In the present embodiment, four through passages 122 arranged side by side at an equal pitch (specifically, a pitch of 90 degrees) are formed around the through passage 121, but the number of the through passages 122 is not limited thereto. The through passages 122 may be one, or may be two, three, or five or more. An opening on the distal end side of each through passage 122 is referred to as a "distal end opening 122A (a second distal end opening)".

The housing portion 13 is formed with a through passage 131 extending in the Z direction and penetrating the housing portion 13. The proximal end of the sheath 11 is inserted into a distal end of the through passage 131. The proximal end of the sheath 11 inserted into the through passage 131 is fixed to an inner wall surface of the through passage 131 by, for example, adhesion.

The endoscopic treatment tool 1 includes a slider 20 (an operation unit). A distal end of the slider 20 is attached to a proximal end of the through passage 131 of the housing portion 13. An outer diameter of the distal end of the slider 20 is slightly smaller than a diameter (an inner diameter) of the through passage 131. Therefore, the slider 20 is slidable in the Z direction inside the through passage 131. Note that a retaining protrusion is formed on an outer peripheral surface of the distal end of the slider 20. Therefore, the slider 20 does not come off the through passage 131.

The slider 20 is formed with a through passage 201 extending in the Z direction and penetrating the slider 20. A proximal end of the tube 30 is inserted into a distal end of the through passage 201. The proximal end of the tube 30 inserted into the through passage 201 is fixed to an inner wall surface of the through passage 201 by, for example, adhesion.

The tube 30 having the proximal end inserted into the through passage 201 of the slider 20 is disposed to extend in the Z direction until reaching a vicinity of the distal end of the sheath 11 through the through passage 131 of the housing portion 13. The tube 30 is an insulating tube made of a resin having flexibility. The tube 30 is made from, for example, PEEK or PTFE.

A side of the housing portion 13 is provided with a plug 40 (the contact portion) connectable to a high-frequency power supply (not illustrated). A proximal end of the plug 40 is connected with a proximal end of a wire 41 as a conductive member. The wire 41 is wired in the tubular portion 10 to the vicinity of the distal end of the sheath 11 through the through passage 131 of the housing portion 13. A distal end of the wire 41 disposed in the vicinity of the distal end of the sheath 11 is electrically connected to a high-frequency knife 50 (a rod-shaped member). The plug 40 and the wire 41 are joined by, for example, a joining material such as a solder, brazing, and laser welding. The wire 41 and the high-frequency knife 50 are also joined by, for example, a joining material such as a solder, brazing, and laser welding.

The high-frequency knife 50 (the rod-shaped member) is inserted through the through passage 121 of the stopper 12. The high-frequency knife 50 is a rod-shaped metal member such as SUS. The high-frequency knife 50 is supplied with a high-frequency current that is from the high-frequency power supply via the plug 40 and the wire 41. With the high-frequency current applied to the high-frequency knife 50, it is possible to perform an incision and exfoliation treatment of tissues in the body cavity.

A proximal end of the high-frequency knife 50 is inserted into a distal end of the tube 30 disposed near the distal end of the sheath 11. An outer diameter of the proximal end of the high-frequency knife 50 is slightly larger than an inner diameter of the tube 30. Therefore, the distal end of the tube 30 is elastically deformed to be in close contact with an outer peripheral surface of the proximal end of the high-frequency knife 50, and in a state of airtightly covering the proximal end of the high-frequency knife 50.

In this way, the high-frequency knife 50 and the slider 20 are mechanically connected to each other via the tube 30 inserted into the through passage 131 of the housing portion 13 and the sheath 11. Since an operator moves the slider 20 to the positive side in the Z direction or the negative side in the Z direction, the tube 30 attached to the through passage 201 of the slider 20 and the high-frequency knife 50 attached to the distal end of the tube 30 integrally move forward and backward in the Z direction. In this way, the high-frequency knife 50 slides in the Z direction within the through passage 121 of the stopper 12, and a protrusion amount from the distal end surface 10A of the stopper 12 changes in response to operations of the operator on the slider 20.

In other words, as the tube 30 moves forward and backward in the Z direction within the tubular portion 10 in response to the operations of the slider 20, the high-frequency knife 50 also moves forward and backward in the Z direction, and the protrusion amount of the high-frequency knife 50 from the distal end surface 10A of the tubular portion 10 changes.

In this way, the stopper 12 as an example of the cap-shaped member is configured to have the through passage 121 slidably supporting the high-frequency knife 50, as an example of the rod-shaped member, in the Z direction. The through passage 121 is an example of the first through passage, and slidably supports the high-frequency knife 50 in the Z direction. The through passage 121 penetrates the stopper 12 in the Z direction, and has an opening on the proximal end side communicating with the sheath 11 and an opening on the distal end side formed in the distal end surface 10A of the stopper 12. Further, the through passages 122 are an example of the second through passage penetrating the stopper 12 in the Z direction, and are formed to extend in the Z direction around the through passage 121. The through passages 122 form a part of the suction channel 70 as an example of the second channel. The opening on the distal end side of each through passage 122 is the distal end opening 122A as an example of the second distal end opening, and is formed in the distal end surface 10A of the stopper 12.

When the slider 20 is moved to the negative side in the Z direction, the high-frequency knife 50 also moves to the negative side in the Z direction. A distal end side surface of the high-frequency knife 50 is formed with a protrusion member 501 protruding laterally over the entire perimeter. An outer diameter of the protrusion member 501 is larger than the diameter (the inner diameter) of the through passage 121 of the stopper 12. Therefore, when the high-frequency knife 50 is moved to the negative side in the Z direction, the protrusion member 501 comes in contact with the distal end surface 10A of the tubular portion 10 (with reference to Fig. 5A and Fig. 8A). In this way, movement of the high-frequency knife 50 toward the negative side in the Z direction is restricted. At this time, the protrusion amount of the high-frequency knife 50 from the distal end surface 10A is minimized.

When the slider 20 is moved to the positive side in the Z direction, the high-frequency knife 50 also moves to the positive side in the Z direction. When the high-frequency knife 50 is moved to the positive side in the Z direction, a step portion 502 formed on an outer peripheral surface of the high-frequency knife 50 comes in contact with a proximal end surface 12A of the stopper 12 (with reference to Fig. 5B). In this way, movement of the high-frequency knife 50 toward the positive side in the Z direction is restricted. At this time, the protrusion amount of the high-frequency knife 50 from the distal end surface 10A of the tubular portion 10 is maximized. Note that the high-frequency knife 50 is configured by covering a proximal end of an inner cylinder formed with a through passage 503 to be described later with an outer cylinder and welding them to each other. One end portion of this outer cylinder forms the step portion 502.

In this way, by operating the slider 20, the operator can change the protrusion amount of the high-frequency knife 50 disposed to protrude from the distal end surface 10A of the tubular portion 10 from the distal end surface 10A. The operator operates the slider 20 according to the content of the procedure (for example, marking, local injection, incision, exfoliation, and hemostasis) and causes the high-frequency knife 50 to protrude from the distal end surface 10A by an appropriate amount.

For example, in the marking treatment, the operator presses the distal end surface 10A against a mucous membrane around a lesion in a state of moving the high-frequency knife 50 backward to a knife backward position (with reference to Fig. 5A and Fig. 8A), and applies a high-frequency current to the high-frequency knife 50. In this way, a marking mark is formed around the lesion.

Note that during various treatments including the marking treatment, it may be difficult to secure a surgical field due to blood or mucus attached to the body cavity. In this case, the operator can secure the surgical field by sucking blood or mucus from a distal end opening 503A formed in the high-frequency knife 50 (or the distal end openings 122A formed in the distal end surface 10A of the tubular portion 10). Note that a specific configuration of the suction channel (one of the first channel and the second channel) provided in the endoscopic treatment tool 1 will be described later.

The local injection is a treatment in which a liquid is locally injected into a submucosa of a lesion to be incised to cause a mucous membrane of the lesion to be incised to float up. In this local injection treatment, the operator moves the high-frequency knife 50 forward to a knife forward position (with reference to Fig. 5B and Fig. 8B) to apply the high-frequency current, and perforates a hole for inserting the high-frequency knife 50 into the submucosa. Next, when the operator inserts the high-frequency knife 50 into the submucosa, a drug solution is injected into the submucosa from the distal end opening 503A (the first distal end opening) of the high-frequency knife 50.

Note that it is also possible to inject the drug solution into the submucosa from the distal end openings 122A formed in the distal end surface 10A of the tubular portion 10. In this case, the operator moves the high-frequency knife 50 forward to the knife forward position (with reference to Fig. 5B and Fig. 8B) to apply the high-frequency current, and perforates a hole for inserting the distal end surface 10A of the tubular portion 10 into the submucosa. Next, the operator inserts the distal end surface 10A into the submucosa in a state of moving the high-frequency knife 50 backward to the knife backward position (with reference to Fig. 5A and Fig. 8A). When inserting the distal end surface 10A into the submucosa, the operator injects the drug solution into the submucosa from the distal end openings 122A.

Note that a specific configuration of the water supply channel (the other of the first channel and the second channel) in the endoscopic treatment tool 1 will be described later.

In the incision treatment, the operator moves the high-frequency knife 50 to the knife forward position (with reference to Fig. 5B and Fig. 8B) while confirming the positions of the high-frequency knife 50 and the marking mark with an endoscope image. In this state, the operator applies the high-frequency current to the high-frequency knife 50, and moves the high-frequency knife 50 along the marking mark to perform an entire perimeter incision. At this time, the protrusion member 501 fits into the mucous membrane and functions as a kind of retainer. Therefore, the high-frequency knife 50 does not inadvertently come out of the mucous membrane during the incision.

In the exfoliation treatment, the operator cauterizes and exfoliates the submucosa of the incised lesion while raising the incised lesion in a state of moving the high-frequency knife 50 forward to the knife forward position (with reference to Fig. 5B and Fig. 8B) to apply the high-frequency current. During the exfoliation treatment, the protrusion member 501 is appropriately caught in the mucous membrane as during the incision treatment. Therefore, the high-frequency knife 50 does not slip and inadvertently come out of the mucous membrane.

In the hemostatic treatment, the operator presses the high-frequency knife 50 against a bleeding ulcer portion or mucous membrane in a state of moving the high-frequency knife 50 backward to the knife backward position (with reference to Fig. 5A and Fig. 8A), and applies the high-frequency current to the high-frequency knife 50 to perform cauterization.

Note that the hemostatic treatment is not limited to a case of stopping the ulcer portion from bleeding after the lesion is exfoliated, and is also performed in a case of stopping a bleeding site during the incision and exfoliation treatment. To remove the blood and the mucus attached to the high-frequency knife 50 by such hemostatic treatment, the operator can clean the high-frequency knife 50 by injecting a cleaning liquid from the distal end openings 122A. Further, it may be difficult to visually recognize the bleeding site due to the blood or the mucus attached to the body cavity. In this case, the operator can suck the blood and the mucus from the distal end openings 122A while spraying the liquid into the body cavity from the distal end opening 503A to remove the blood and the mucus. In this way, the operator can easily visually recognize the site where bleeding is to be stopped. Note that alternatively, the liquid may be sprayed into the body cavity from the distal end openings 122A to remove the blood and the mucus, and the blood and the mucus may be sucked from the distal end opening 503A.

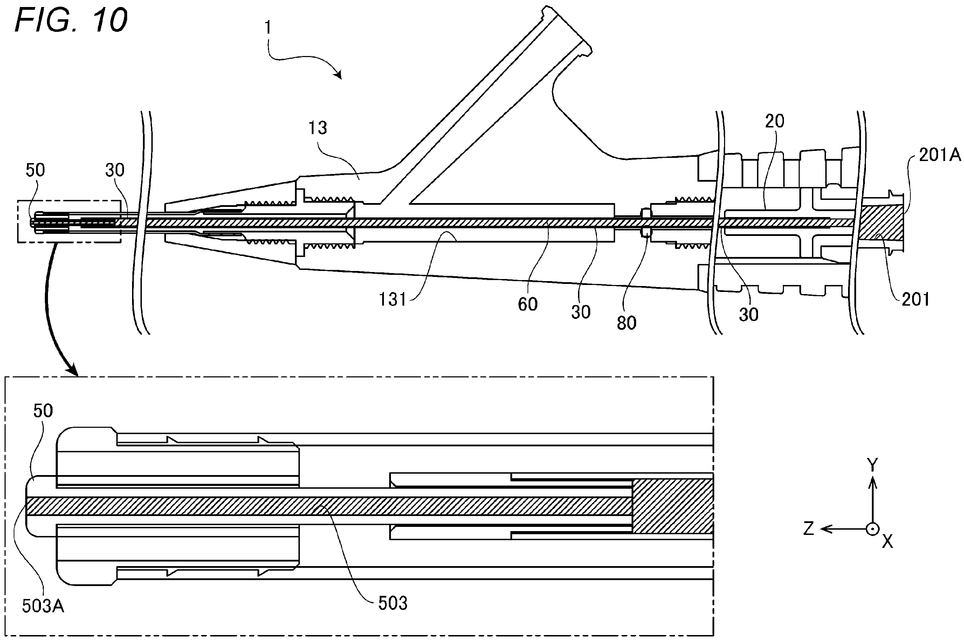

A specific configuration of the water supply channel 60 provided in the endoscopic treatment tool 1 will be described. Fig. 10 is a cross-sectional view of the endoscopic treatment tool 1 illustrating the water supply channel 60. In Fig. 10, a region indicated by a hatching is the water supply channel 60. As illustrated in Fig. 10, the water supply channel 60 is composed of the through passage 201 formed in the slider 20, the tube 30, and the through passage 503 formed in the high-frequency knife 50. The through passage 503 is a through passage extending in the Z direction to penetrate the high-frequency knife 50.

A proximal end of the through passage 201 of the slider 20 is a connection port 201A (a first connection port) to which a tool is connectable. A tool connectable to the connection port 201A is a tool for water supply or suction, and is, for example, a water supply pump, a suction pump, or a syringe.

As illustrated in Fig. 10, the through passage 201 of the slider 20 communicates with the tube 30, and the tube 30 communicates with the through passage 503 of the high-frequency knife 50. Therefore, for example, in a case where a water supply pump is connected to the connection port 201A, the cleaning liquid or the like supplied to the through passage 201 via the connection port 201A flows through the tube 30 and the through passage 503, and injected to the outside from the distal end opening 503A (an opening on the distal end side of the through passage 503) formed in the distal end surface of the high-frequency knife 50. In this way, it is possible to inject the drug solution into the submucosa or remove attachments (the blood or the mucus).

Note that by connecting a suction pump to the connection port 201A, the water supply channel 60 can function as a suction channel. In this case, for example, the attachments such as the blood or the mucus attached to the body cavity are sucked into the through passage 503 via the distal end opening 503A. The attachments sucked into the through passage 503 are put into, for example, a container included in the suction pump via the tube 30 and the through passage 201.

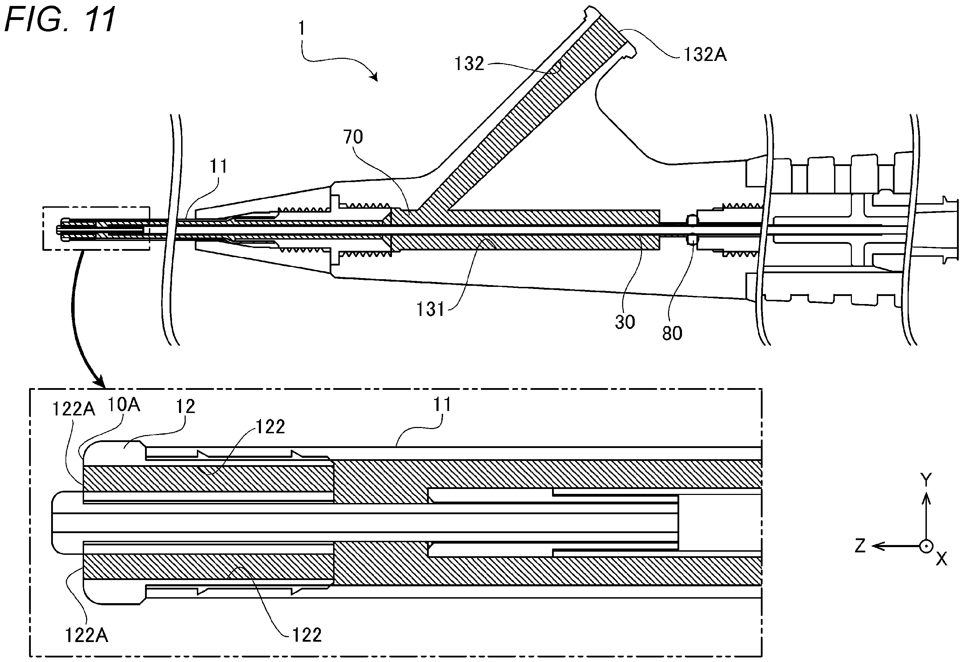

A specific configuration of the suction channel 70 provided in the endoscopic treatment tool 1 will be described. Fig. 11 is a cross-sectional view of the endoscopic treatment tool 1 illustrating the suction channel 70. In Fig. 11, a region indicated by a hatching is the suction channel 70.

Here, a through passage 132 branching the through passage 131 obliquely upward is formed in the housing portion 13. A proximal end of the through passage 132 branched from the through passage 131 is a connection port 132A (a second connection port) to which a tool is connectable. Similarly to the connection port 201A of the slider 20, the connection port 132A can be connected with a tool for water supply or suction.

As illustrated in Fig. 11, the suction channel 70 is composed of the through passages 131 and 132, the sheath 11, and a plurality of the through passages 122 formed in the stopper 12. More specifically, the tube 30 forming a part of the water supply channel 60 passes through the through passage 131 and the sheath 11. Therefore, in the through passage 131, a channel defined by the inner wall surface of the through passage 131 and an outer peripheral surface of the tube 30 forms a part of the suction channel 70. Further, in the sheath 11, a channel defined by an inner peripheral surface of the sheath 11 and the outer peripheral surface of the tube 30 forms a part of the suction channel 70.

As illustrated in Fig. 11, the through passage 132 communicates with the through passage 131, the through passage 131 communicates with the sheath 11, and the sheath 11 communicates with each of the through passages 122. Therefore, for example, in a case where the suction pump is connected to the connection port 132A, the attachments in the body cavity are sucked into the through passages 122 through the distal end openings 122A formed in the distal end surface 10A of the stopper 12. The attachments sucked into the through passages 122 are put into, for example, a container included in the suction pump via the sheath 11 and the through passages 131 and 132.

Note that by connecting the water supply pump to the connection port 132A, the suction channel 70 can function as a water supply channel. In this case, the cleaning liquid or the like supplied to the through passage 132 through the connection port 132A flows through the through passage 131, the sheath 11, and the through passages 122, and injected to the outside from each distal end opening 122A. In this way, it is possible to inject the drug solution into the submucosa or remove the attachments.

In this way, by providing two separate channels in the endoscopic treatment tool 1, it is possible to perform water supply or suction without exchanging the pumps. Further, both the water supply and the suction can be performed simultaneously. For example, various treatments such as local injection can be performed while the suction pump is constantly operated to constantly suck blood and mucus. In other words, by adopting a configuration in which the water supply and the suction are performed simultaneously, it is possible to constantly secure a good surgical field during ESD treatment.

Further, when the high-frequency current is applied to the high-frequency knife 50, the attachments, for example, hemoglobin, protein or the like thermally clotted by cauterization, may be attached to a portion of the high-frequency knife 50 in contact with the mucous membrane. For example, by performing various treatments such as local injection while constantly operating the suction pump and constantly sucking the blood and the mucus around the high-frequency knife 50, the above attachments can be reduced. In other words, by adopting a configuration in which the water supply and the suction can be performed simultaneously, it is possible to reduce a thermally clotted product that may be attached to the high-frequency knife 50 during the ESD treatment.

It is necessary to form a slight gap between the inner wall surface of the through passage 131 and the outer peripheral surface of the tube 30 disposed in the through passage 131 such that the tube 30 can move forward and backward in the Z direction when the slider 20 is operated. However, when such a gap exists, airtightness of the through passage 131 inside the tubular portion 10 cannot be secured. Accordingly, in the present embodiment, an O-ring 80 (a seal member) airtightly sealing the gap is fitted to the outer periphery of the tube 30. In other words, the O-ring 80 securing airtightness of the suction channel 70 inside the tubular portion 10 is disposed on an outer periphery of a portion of the water supply channel 60 passing through the suction channel 70 (an outer periphery of a portion of the tube 30 passing through the through passage 131).

Note that by fitting the O-ring 80, a frictional resistance is generated between the outer peripheral surface of the tube 30 and the O-ring 80. This frictional resistance causes the slider 20 to move in the Z direction with an appropriate load when operated. Therefore, it is easy for the operator to move the slider 20 slightly, and adjust the protrusion amount of the high-frequency knife 50.

As illustrated in Fig. 9, in the front view of the distal end surface 10A of the tubular portion 10, the high-frequency knife 50 is located in the central region of the distal end surface 10A. Further, four distal end openings 122A are located side by side at an equal pitch in a peripheral region of the distal end surface 10A located outside this central region (that is, the periphery of the high-frequency knife 50). In this way, the high-frequency knife 50 and the distal end openings 122A are located without overlapping each other in the front view, and for example, even in a case where the high-frequency knife 50 is moved backward to the knife backward position (with reference to Fig. 5A and Fig. 8A), the distal end openings 122A are not closed by the high-frequency knife 50. Therefore, both the water supply and the suction can be performed constantly.

As illustrated in Fig. 9, since an outer periphery of the protrusion member 501 and the distal end openings 122A are disposed in a relationship in which they are in contact with each other in the front view, it is possible to secure a large diameter of each distal end opening 122A (and each through passage 122) while preventing the distal end openings 122A from being closed by the high-frequency knife 50.

Note that even when the distal end openings 122A are closed more or less by the high-frequency knife 50, both the water supply and the suction can be performed simultaneously. In order to further increase the diameter of each distal end opening 122A (and each through passages 122), a part of the distal end opening 122A may be disposed and configured to overlap with the high-frequency knife 50 in the front view (that is, a part of the distal end opening 122A is hidden by the protrusion member 501 in the front view).

Exemplary embodiments of the present invention are described above. The embodiments of the present invention are not limited to those described above, and various modifications can be made within the scope of the technical idea of the present invention. For example, contents in appropriate combination with the embodiments illustratively clarified in the description or obvious embodiments are also included in the embodiments of the present invention.

In the above embodiments, the cross-sectional shapes of the through passages 122 of the stopper 12 and the through passage 503 of the high-frequency knife 50 in an axis-orthogonal direction are substantially perfect circles, but the shapes of the through passages 122 and 503 are not limited thereto. The cross-sectional shapes of the through passages 122 and 503 in the axis-orthogonal direction may be other shapes such as an ellipse or a prism.

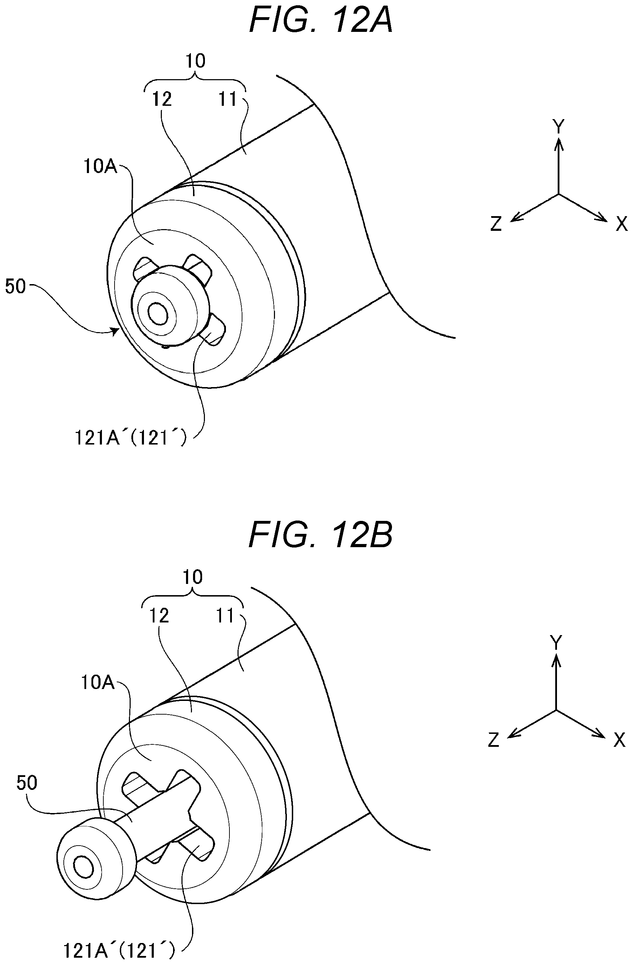

The endoscopic treatment tool 1 according to the modifications of the present invention will be described with reference to Fig. 12A and Fig. 12B and Fig. 13 to Fig. 15.

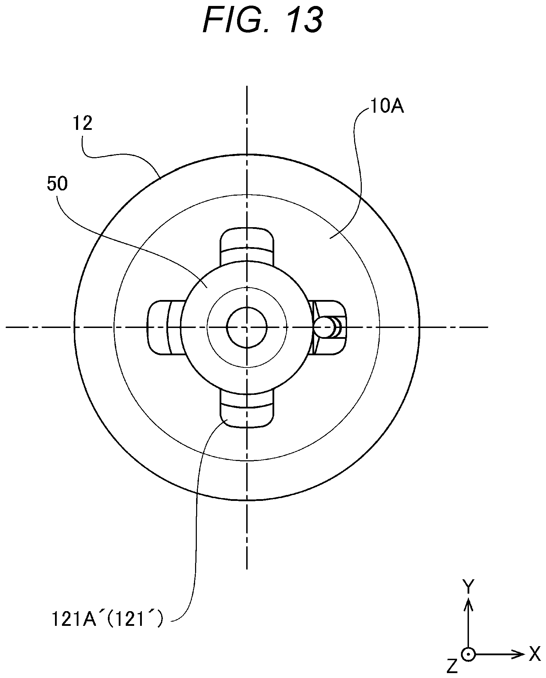

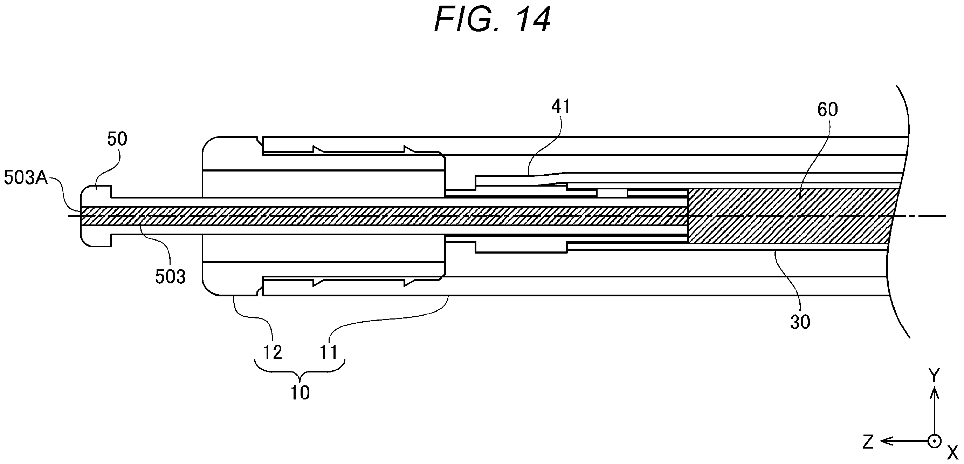

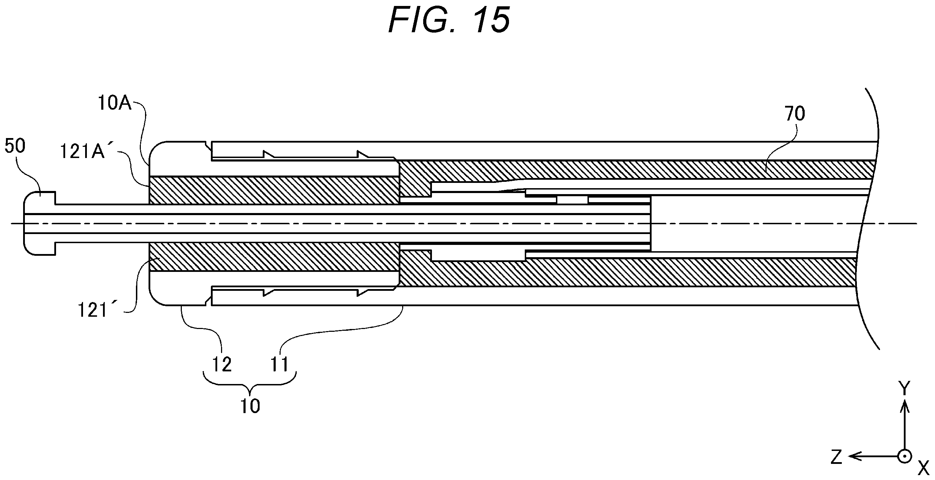

Fig. 12A and Fig. 12B are views similar to Fig. 8A and Fig. 8B, respectively, and are perspective views of the distal end of the tubular portion 10 according to the present modification. Fig. 13 is a view similar to Fig. 9, and is a view when the distal end surface 10A of the tubular portion 10 according to the present modification is viewed from the positive side in the Z direction (that is, a front view of the distal end surface 10A). Fig. 14 is a view similar to Fig. 10, and is a cross-sectional view of the endoscopic treatment tool 1 illustrating the water supply channel 60 according to the present modification. Fig. 15 is a view similar to Fig. 11, and is a cross-sectional view of the endoscopic treatment tool 1 illustrating the suction channel 70 according to the present modification.

Note that in the present modification, descriptions overlapping with those of the above embodiments will be omitted as appropriate. Further, in the present modification, the configurations of the water supply channel 60 and the suction channel 70 behind the distal end of the tubular portion 10 are substantially the same as those in the above embodiments. Therefore, in Fig. 14 and Fig. 15, only the water supply channel 60 and the suction channel 70 at the distal end portion of the tubular portion 10 are illustrated, respectively.

In the present modification, a through passage 121' of the stopper 12 is an example of the support passage slidably supporting the high-frequency knife 50, being an example of the rod-shaped member, in the Z direction, and forms a part of the suction channel 70 as an example of the second channel (with reference to, for example, Fig. 12B, Fig. 14, and Fig. 15). As illustrated in Fig. 14 and Fig. 15, the through passage 121' is also an example of the third through passage penetrating the stopper 12 in the Z direction, and has an opening on a proximal end side communicating with the sheath 11 and an opening on a distal end side formed in the distal end surface 10A of the stopper 12. The opening on the distal end side of the through passage 121' is referred to as a "distal end opening 121A'".

More specifically, the through passage 121' is a single through passage formed in a plus shape (that is, a shape like +) in an XY cross section. The high-frequency knife 50 is disposed to penetrate the through passage 121' in the Z direction at a portion where a portion extending in the X direction and a portion extending in the Y direction intersect in the XY cross section (that is, a center region of the through passage 121' in the XY cross section).

In the present modification, similarly to the above embodiments, the water supply channel 60 is composed of the through passage 201 formed in the slider 20, the tube 30, and the through passage 503 formed in the high-frequency knife 50.

However, the configuration of the suction channel 70 is different from that in the above embodiments. In the present modification, the suction channel 70 is composed of the through passages 131 and 132 of the housing portion 13, the sheath 11, and the through passage 121'. Therefore, for example, in a case where the suction pump is connected to the connection port 132A, the attachments in the body cavity are sucked into the through passage 121' through the distal end opening 121A' formed in the distal end surface 10A of the stopper 12. The attachments sucked into the through passage 121' are put into, for example, a container included in the suction pump via the sheath 11 and the through passages 131 and 132.

In the present modification, since the through passage 121' slidably supporting the high-frequency knife 50 in the Z direction is configured to also serve as a part of the suction channel 70, for example, the outer diameter of the tubular portion 10 can be suppressed to be smaller than those in the configurations of the above embodiments (that is, a configuration in which the through passages 122 is formed around the through passage 121). Further, it is possible to increase a volume of the suction channel 70 at the distal end portion of the tubular portion 10. By increasing the volume of the suction channel 70, for example, the amounts of the attachments capable of being sucked per unit time can be increased.

## Claims

1. An endoscopic treatment tool, comprising:
a tubular portion;
a rod-shaped member that is disposed to protrude from a distal end surface of the tubular portion, and has a variable protrusion amount from the distal end surface in response to operations of an operation unit;
a first connection port to which a tool for water supply or suction is connectable;
a first distal end opening that is formed in a distal end surface of the rod-shaped member;
a first channel that is formed in the tubular portion to extend in an axial direction of the tubular portion, and has a distal end communicating with the first distal end opening and a proximal end communicating with the first connection port;
a second connection port to which the tool is connectable;
a second distal end opening that is formed in the distal end surface of the tubular portion; and
a second channel that is formed in the tubular portion to extend in the axial direction, and has a distal end communicating with the second distal end opening and a proximal end communicating with the second connection port.

2. The endoscopic treatment tool according to claim 1, further comprising:
a tube that communicates with a through passage formed in the rod-shaped member, wherein
the first distal end opening is an opening of the through passage formed in the distal end surface of the rod-shaped member, and
the first channel includes the through passage and the tube.

3. The endoscopic treatment tool according to claim 2, wherein
a proximal end of the rod-shaped member is inserted into a distal end of the tube to allow the through passage to communicate with the tube, and
as the tube moves forward and backward in the axial direction within the tubular portion in response to the operations of the operation unit, the rod-shaped member also moves forward and backward in the axial direction, and the protrusion amount of the rod-shaped member from the distal end surface of the tubular portion changes.

4. The endoscopic treatment tool according to any one of claims 1 to 3, wherein
in a front view of the distal end surface of the tubular portion, the rod-shaped member is located in a central region of the distal end surface of the tubular portion, and the second distal end opening is located in a peripheral region of the distal end surface of the tubular portion located outside the central region.

5. The endoscopic treatment tool according to claim 4, wherein
a plurality of the second distal end openings are formed in the distal end surface of the tubular portion, and
the plurality of the second distal end openings are located side by side at an equal pitch around the rod-shaped member in the front view.

6. The endoscopic treatment tool according to claim 4 or 5, wherein
the tubular portion includes
a sheath; and
a cap-shaped member attached to the sheath to close a distal end opening of the sheath, while
the cap-shaped member includes
a first through passage that slidably supports the rod-shaped member in the axial direction, has an opening on a proximal end side communicating with the sheath and an opening on a distal end side formed in the distal end surface of the cap-shaped member, and penetrates the cap-shaped member; and
a second through passage that is formed to extend in the axial direction around the first through passage and penetrates the cap-shaped member,
the second through passage forms a part of the second channel, and
an opening on the distal end side of the second through passage is the second distal end opening, and is formed in a distal end surface of the cap-shaped member.

7. The endoscopic treatment tool according to any one of claims 1 to 3, wherein
the tubular portion includes a support passage that slidably supports the rod-shaped member in the axial direction, and
the support passage forms a part of the second channel.

8. The endoscopic treatment tool according to claim 7, wherein
the tubular portion includes
a sheath; and
a cap-shaped member attached to the sheath to close a distal end opening of the sheath,
the support passage is a third through passage that penetrates the cap-shaped member, and has an opening on a proximal end side communicating with the sheath and an opening on a distal end side formed in a distal end surface of the cap-shaped member, and
the third through passage forms a part of the second channel.

9. The endoscopic treatment tool according to any one of claims 1 to 8, wherein
at least a part of the first channel passes through the second channel, and
a seal member securing airtightness of the second channel inside the tubular portion is disposed on an outer periphery of a portion of the first channel passing through the second channel.

10. The endoscopic treatment tool according to any one of claims 1 to 9, further comprising:
a contact portion to which a high-frequency power supply for allowing a high-frequency current to flow is connectable; and
a wire having conductivity disposed in the tubular portion, wherein
the rod-shaped member has conductivity, and
the wire has a distal end electrically connected to the rod-shaped member, and a proximal end electrically connected to the contact portion.
